# EUROPÄISCHE PATENTSCHRIFT

(11) **EP 3 476 948 B1**
(45) Veröffentlichungstag und Bekanntmachung des Hinweises auf die Patenterteilung: **21.01.2026**
(21) Anmeldenummer: 18193967.9
(22) Anmeldetag: 12.09.2018
(51) Int. Cl.: C12Q 1/6881

(54) **VERFAHREN UND VORRICHTUNG ZUR BESTIMMUNG DER BLUTGRUPPE EINER KATZE IM AB-BLUTGRUPPENSYSTEM**
METHOD AND DEVICE FOR DETERMINING THE BLOOD GROUP OF A CAT IN AB BLOOD GROUP SYSTEM
PROCÉDÉ ET DISPOSITIF DE DÉTERMINATION DU GROUPE SANGUIN D'UN CHAT DANS LE SYSTÈME DE GROUPE SANGUIN ABO

(30) Priorität: 25.10.2017 DE 102017124998
(43) Veröffentlichungstag der Anmeldung: 01.05.2019
(73) Patentinhaber: LABOklin Labor für klinische Diagnostik GmbH & Co. KG, 97688 Bad Kissingen (DE)
(72) Erfinder: Giger, Urs, Pennsylvania 19063 (US); Heimberger, Kevin, 97218 Gerbrunn (DE); Kehl, Alexandra, 97702 Münnerstadt (DE); Langbein-Detsch, Ines, 97688 Bad Kissingen (DE)
(74) Vertreter: advotec.

(56) Entgegenhaltungen:
- KR-B1- 101 684 832
- US-A1- 2008 220 427
- BARBARA GANDOLFI ET AL: "A Novel Variant in CMAH Is Associated with Blood Type AB in Ragdoll Cats", PLOS ONE, vol. 11, no. 5, 12 May 2016 (2016-05-12), pages e0154973, XP055550402, DOI: 10.1371/journal.pone.0154973
- TOSHINORI OMI ET AL: "Molecular Characterization of the Cytidine Monophosphate-N-Acetylneuraminic Acid Hydroxylase (CMAH) Gene Associated with the Feline AB Blood Group System", PLOS ONE, vol. 11, no. 10, 18 October 2016 (2016-10-18), pages e0165000, XP055550397, DOI: 10.1371/journal.pone.0165000
- BARBARA BIGHIGNOLI ET AL: "Cytidine monophospho-N-acetylneuraminic acid hydroxylase (CMAH) mutations associated with the domestic cat AB blood group", BMC GENETICS, vol. 8, no. 1, 1 June 2007 (2007-06-01), GB, pages 27 - 27, XP055550399, ISSN: 1471-2156, DOI: 10.1186/1471-2156-8-27
- DATABASE EMBL [online] 10 July 2007 (2007-07-10), "Felis catus cytidine monophospho-N-acetylneuraminic acid hydroxylase (CMAH) mRNA, CMAH-A allele, complete cds.", retrieved from EBI accession no. EM_STD:EF127684 Database accession no. EF127684
- S. TASKER ET AL: "Feline blood genotyping versus phenotyping, and detection of non-AB blood type incompatibilities in UK cats", JOURNAL OF SMALL ANIMAL PRACTICE., vol. 55, no. 4, 1 April 2014 (2014-04-01), GB, pages 185 - 189, XP055550392, ISSN: 0022-4510, DOI: 10.1111/jsap.12180
- ALEXANDRA KEHL ET AL: "Molecular characterization of blood type A, B, and C (AB) in domestic cats and a CMAH genotyping scheme", PLOS ONE, vol. 13, no. 9, 20 September 2018 (2018-09-20), pages e0204287, XP055550378, DOI: 10.1371/journal.pone.0204287

## Beschreibung

Die Erfindung betrifft ein Verfahren zur genotypischen Bestimmung der Blutgruppe einer Katze im AB-Blutgruppensystem nach dem Oberbegriff der unabhängigen Ansprüche.

Die Erstbeschreibung des felinen Blutgruppensystems erfolgte bereits im Jahre 1915. Dennoch dauerte es noch einige Jahre, bis das heute bei Katzen gebräuchliche AB-Blutgruppensystem 1981 als dieses charakterisiert wurde. So können bei Katzen (*Felidae*) drei verschiedene serologische Blutgruppen, nämlich A, B und C, unterschieden werden, wobei die Blutgruppe A die mit Abstand am häufigsten auftretende unter den drei Blutgruppen ist. In der momentan vorherrschenden Literatur findet man die Blutgruppe C meist unter der irreführenden Bezeichnung AB.

Aus dem Stand der Technik ist es beispielsweise bekannt, dass der phänotypische Unterschied der drei Blutgruppen auf ein einziges Antigen zurückzuführen ist, welches in die Zellmembran der Erythrozyten eingelagert ist. Die N-Glycolylneuraminsäure (NeuGc), eine Sialinsäure, ist auf Erythrozyten von Katzen der Blutgruppe A zu finden, wohingegen bei Katzen der Blutgruppe B eine Vorstufe des Antigens, die Sialinsäure N-Acetylneuraminsäure (NeuAc), exprimiert wird. Die Erythrozyten im Blut von C-Katzen besitzen beide Formen der Moleküle. Diese Epitope dienen als Antigene für blutgruppenspezifische Antikörper der jeweils anderen Blutgruppe. Somit besitzen Katzen der Blutgruppe B Antikörper gegen die N-Glycolylneuraminsäure von A-Katzen und reziprok dazu besitzen die Katzen der Blutgruppe A Antikörper gegen die N-Acetylneuraminsäure der B-Katzen. C-Katzen besitzen keinen der beiden Antikörper, da auf ihren Erythrozyten beide Antigene vorkommen und die Präsenz eines der beiden Antikörper zu einer Autoimmunreaktion führen würde.

Das AB-Blutgruppensystem ist nicht nur in den verschiedenen Rassen der Hauskatze konserviert, sondern ebenfalls in Wildkatzen und anderen Feliden. Untersuchungen aus dem Jahr 1999 zeigten, dass von Puma (*Puma concolor*) über Rotluchs (*Lynx rufus*) bis hin zum Geparden (*Acinonyx jubatus*) alle dasselbe Blutgruppensystem aufweisen. Durch Untersuchungen mit Kreuzreaktionen konnte hier darauf geschlossen werden, dass die Antikörper und Antigene der verschiedenen Feliden dieselben oder zumindest sehr ähnlich sind.

Die Notwendigkeit der Typisierung von Katzen liegt darin begründet, dass bekannter Weise die Reaktion von Antigen und Antikörper zu einer Hämolyse und/oder einer Hämagglutination führt, weswegen eine Feststellung der serologischen Blutgruppe vor einer Bluttransfusion unabdingbar ist. Eine Immunreaktion kann nach einer A-B inkompatiblen Bluttransfusion auftreten.

Doch nicht nur bei der Transfusion, sondern auch in der Zucht besteht die Notwendigkeit, die Blutgruppen zu beachten. Katzen der Blutgruppe A besitzen relativ schwache Alloantikörper gegen die Antigene der Blutgruppe B, auch ist der Antikörpertiter meistens relativ gering. B-Katzen hingegen bilden sehr starke Antikörper in hohen Konzentrationen aus. Diese können bei der Zucht eine hohe Sterberate bei Katzenwelpen auslösen, die sogenannte Neonatale Isoerythrolyse. Die Antikörper gegen die A-Antigene finden sich in hoher Konzentration in der Muttermilch wieder. Hat eine Mutterkatze der Blutgruppe B Kitten der Blutgruppe A oder C, so nehmen diese mit der Muttermilch die Antikörper auf, was in den meisten Fällen zum Tod der Katzenwelpen führt.

Die genetische Ursache für die Ausprägung der Blutgruppen bei Katzen war jedoch lange Zeit unbekannt, bis im Jahre 2007 diverse Mutationen in dem Gen *Cytidin-Monophosphat-N-Acetylneuraminsäure-Hydroxylase* (*CMAH*) als Auslöser aufgefunden wurde. Allerdings gibt es bis dato noch keine Beweise dafür, dass diese genetischen Varianten tatsächlich für die Aktivität des Enzyms ursächlich sind. Dieses Gen codiert für das Enzym Cytidin-Monophosphat-N-Acetylneuraminsäure-Hydroxylase, eine Oxidoreduktase, welche die Reaktion von N-Acetylneuraminsäure (NeuAc) zu N-Glycolylneuraminsäure (NeuGc) katalysiert. Das Enzym besitzt im reaktiven Zentrum sowohl ein Rieske-Typ-(2Fe-2S)-Cluster als auch ein nicht-Häm-Eisenion, wobei es aus dem Stand der Technik bekannt ist, dass Mutationen an diesen Stellen die wahrscheinlichste Ursache für einen Funktionsverlust des Enzyms sind. Ein solcher scheint mit der Ausbildung der Blutgruppe B assoziiert zu sein. Wenn das CMAH-Enzym so stark mutiert ist, dass es seine Funktion nicht mehr erfüllen kann, so wird NeuAc nicht mehr zu NeuGc umgesetzt und man erhält den Phänotyp der Blutgruppe B. Aus der Dominanz des A-Allels für Blutgruppe A lässt sich schließen, dass selbst eine verminderte Enzymkonzentration des funktionsfähigen CMAH ausreicht, um nahezu die gesamte NeuAc zu NeuGc umzusetzen und somit den Phänotyp der Blutgruppe A herbeizuführen.

Es ist daher nicht verwunderlich, dass auf dem Stand der Technik zahlreiche Schnelltests zur Bestimmung der Blutgruppe einer Katze bekannt sind. So ist es beispielsweise bekannt, dass bei einem Schnelltest auf einer Testmembran an festgelegten Stellen monoklonale Antikörper gegen spezifische Antigene auf den Erythrozyten der Blutgruppen fixiert sind. Wandern die mit dem Puffer behandelten Erythrozyten nun die Membran entlang, treffen diese auf die Antikörper und es kann eine Agglutination ausgelöst werden. Nachteilig bei den zuvor genannten Schnelltests der Phänotypen ist es, dass diese nicht spezifisch sind und sich somit die erhaltenen Ergebnisse teils widersprechen. Dies liegt darin begründet, dass bislang keine eindeutige, genetische Ursache für die phänotypische Ausprägung der Blutgruppen festgestellt werden konnte. Es ist des Weiteren von Nachteil, dass mit den derzeit verfügbaren Verfahren und Vorrichtungen keine eindeutige Bestimmung der Blutgruppen der Katze möglich ist.

Es ist des Weiteren bekannt, dass ein Forward- und Backward-Typing durchzuführen. Sowohl beim Forward- als auch beim Back-Typing macht man es sich zunutze, dass im Blut von B-Katzen Antikörper gegen Epitope auf Erythrozyten von A-Katzen vorhanden sind, die eine Hämagglutination auslösen können. Bringt man nun A-Erythrozyten mit B-Serum/Plasma zusammen, so findet eine Agglutination statt, was umgekehrt aufgrund der niedrigen Antikörper-Titer von A-Katzen nicht oder nur sehr begrenzt funktioniert. Ein weiterer Nachteil dieser Bestimmung liegt darin begründet, dass aufgrund des geringen Titers und der geringen Effektivität der Anti-B-Antikörper im Serum von A-Katzen gegebenenfalls nur eine schwache Agglutination von A-Serum mit B-Erythrozyten zu erwarten ist. Zudem ist keine eindeutige Bestimmung der Blutgruppe C bei der Katze möglich.

Die Veröffentlichung von GANDOLFI, B. et al. aus PLoS One (2016) 11 (5):e0154973, Seiten 1-15 mit dem Titel "A Novel Variant in CMAH Is Associated with Blood Type AB in Ragdoll Cats" offenbart eine Vielzahl verschiedener Mutationen oder Deletionen im felinen CMAH-Gen zur Bestimmung der Blutgruppe einer Katze im AB-Blutgruppensystem.

In der Veröffentlichung OMI, T et al. aus PLoS One (2016) 11 (10):e0165000, Seite 1-17 mit dem Titel "Molecular Characterization of the Cytidine Monophospate-N-Acetylneuraminic Acid Hydroxylase (CMAH) Gene Associated with the Feline AB Blood Group System" ist eine Vielzahl verschiedener Mutationen im felinen CMAH-Gen offenbart, welche mit den Blutgruppen A, B oder C bei unterschiedlichen Katzen assoziiert werden.

Auch die Veröffentlichung von BIGHIGNOLI et al. aus BMC GENETICS (2007) 8 (1) mit dem Titel "Cytidine monophospho-N-acetylneuraminic acid hydroxylase (CMAH) mutations associated with the domestic cat AB blood group" und die Veröffentlichung von TASKER et al.: "Feline blood genotyping versus phenotyping, and detection of non-AB blood type incompatibilities in UK cats", JOURNAL OF SMALL ANIMAL PRACTICE, Bd. 55, Nr. 4, 1. April 2014 offenbaren eine Vielzahl verschiedener Mutationen im felinen CMAH-Gen zur Bestimmung der Blutgruppe einer Katze im AB-Blutgruppensystem.

Die Offenlegungsschrift US 2008 / 0 220 427 A1 befasst sich mit unterschiedlichen Katzenrassen, sowie deren Frequenzen und deren Ausprägung für die Blutgruppen A, B und C. Auch in dieser Veröffentlichung werden verschiedene Mutationen im felinen CMAH-Gen als ursächlich für die Blutgruppen A, B und C genannt.

Es besteht daher ein großer Bedarf an einem Verfahren zur genotypischen Bestimmung der Blutgruppe einer Katze, mittels welchen eine schnelle, zuverlässige, einfache und hinreichend genaue Bestimmung und/oder Unterscheidung der drei für Felidae bekannten Blutgruppen im AB-Blutgruppensystem gewährleistet ist. Zudem sollte das Verfahren kostengünstig realisierbar, zuverlässig arbeitend und auftretende Besonderheiten einer bestimmten Unterfamilie und/oder Rasse der Felidae berücksichtigen. Ein weiterer Aspekt besteht darin, dass das Verfahren praktisch ohne weitere Anleitung durchführbar und die Interpretation klar sind. Die Erfindung hat sich daher die Aufgabe gestellt, ein Verfahren zur genotypischen Bestimmung der Blutgruppe einer Katze im AB-Blutgruppensystem bereitzustellen, um die oben genannten Schwierigkeiten zu überwinden, die Diagnostik zu verbessern und um die Belastung für die zu untersuchende Katze und die für die Durchführung des Verfahrens entstehenden Kosten auf ein Minimum zu reduzieren.

Diese Aufgabe wird auf überraschend einfache, aber wirkungsvolle Weise durch Verfahren zur genotypischen Bestimmung der Blutgruppe einer Katze im AB-Blutgruppensystem nach der Lehre der unabhängigen Ansprüche gelöst.

Erfindungsgemäß ist ein Verfahren zur genotypischen Bestimmung der Blutgruppe einer Katze im AB-Blutgruppensystem vorgeschlagen, welches die folgenden Schritte umfasst:
a) Ermitteln der Anwesenheit oder Abwesenheit von mindestens drei Mutationen in einem von beiden Allelen oder in beiden Allelen des felinen Gens Cytidin-Monophosphat-N-Acetylneuraminsäure-Hydroxylase in einer biologischen Probe aus der Katze, wobei die Mutationen eine G zu T Substitution an Position 179, eine T zu A Substitution an Position 268 und eine T-Deletion an Position 1322sind; und
b) Bestimmen der Blutgruppe der Katze, wobei die Anwesenheit von einer der Mutationen in einem von beiden Allelen und/oder die Abwesenheit von mindestens einer der Mutationen in beiden Allelen indikativ für die Blutgruppe A ist oder wobei die Anwesenheit von mindestens einer der Mutationen in beiden Allelen und/oder die Anwesenheit von mindestens zwei der Mutationen in einem von beiden Allelen indikativ für die Blutgruppe B ist.

Erfindungsgemäß ist weiterhin ein Verfahren zur genotypischen Bestimmung der Blutgruppe einer Katze im AB-Blutgruppensystem vorgeschlagen, welches die folgenden Schritte umfasst:
a) Ermitteln der Anwesenheit oder Abwesenheit von vier Mutationen in einem von beiden Allelen oder in beiden Allelen des felinen Gens *Cytidin-Monophosphat-N-Acetylneuraminsäure-Hydroxylase* (*CMAH*) in einer biologischen Probe aus der Katze, wobei die Mutationen eine G zu T Substitution an Position 179, eine T zu A Substitution an Position 268, eine C zu T Substitution an Position 364 und eine T-Deletion an Position 1322 sind; und
b) Bestimmen der Blutgruppe der Katze, wobei die Anwesenheit von der Mutation an Position 364 in beiden Allelen oder von der Anwesenheit von der Mutation an Position 364 in einem Allel und von mindestens einer der Mutationen ausgewählt aus der Gruppe umfassend die Positionen 179, 268 und 1322 in einem von beiden Allelen oder in beiden Allelen indikativ für die Blutgruppe C ist.

Das erfindungsgemäße Verfahren beruht auf dem Grundgedanken, dass es zur zuverlässigen, schnellen, einfachen, hinreichend genauen und genotypischen Bestimmung der Blutgruppe, und damit der Unterscheidung aller drei bei Katzen bekannten Blutgruppen im AB-Blutgruppensystem voneinander, ausreichend ist, die Anwesenheit oder Abwesenheit mindestens dreier Mutationen in einem von beiden Allelen oder in beiden Allelen des felinen Gens *Cytidin-Monophosphat-N-Acetylneuraminsäure-Hydroxylase* (*CMAH*) in einer biologischen Probe der Katze zu ermitteln. Im Rahmen der Erfindung ist es dabei erkannt worden, dass es zur genotypischen Bestimmung der Blutgruppe der Katze im AB-Blutgruppensystem ausreichend ist, die Anwesenheit, das heißt die Nachweisbarkeit in einem von beiden Allelen oder in beiden Allelen, oder Abwesenheit, das heißt die Nicht-Nachweisbarkeit in beiden Allelen, von mindestens drei Mutation in einem von beiden Allelen oder in beiden Allelen des *CMAH*-Gens in der biologischen Probe der Katze zu ermitteln, wobei die Mutationen eine G zu T Substitution an Position 179, eine T zu A Substitution an Position 268 und eine T-Deletion an Position 1322 sind bzw. wobei die Mutationen eine G zu T Substitution an Position 179, eine T zu A Substitution an Position 268, eine C zu T Substitution an Position 364 und eine T-Deletion an Position 1322 sind. Bevorzugt wird die Anwesenheit oder Abwesenheit von 3, 4, 5, 6, 7, 8, 9, 10, 11, 12, 13, 14, 15, 16 oder mehr Mutationen in dem Gen *CMAH* ermittelt.

Der Begriff *"Cytidin-Monophosphat-N-Acetylneuraminsäure-Hydroxylase* (*CMAH*)" betrifft ein Gen, welches für das Enzym Cytidin-Monophosphat-N-Acetylneuraminsäure-Hydroxylase, eine Oxidoreduktase, codiert. Dieses Enzym katalysiert die Reaktion von N-Acetylneuraminsäure (NeuAc) zu N-Glycolylneuraminsäure (NeuGc). Das Gen *CMAH* setzt sich aus 16 Exons zusammen, wobei zwei Isoformen des Proteins existieren, die beide notwendig sind, um die katalytische Aktivität zu gewährleisten. Die Isoformen CMAH 1a und CMAH 1b entstehen durch unterschiedliches Splicing der Leader-Exons. Bei CMAH 1a wird das Exon 1a an das Exon 2 gespliced, wohingegen bei CMAH 1b dementsprechend das Exon 1b an das Exon 2 gespliced wird. In Rahmen der Erfindung beginnt die Zählung bevorzugt beim Adenosin des Startcodons ATG, welches sich im Exon 1a befindet, wie in SEQ ID NO: 1 für die Nukleinsäure-Sequenz und in SEQ ID NO: 2 für die entsprechende Aminosäure-Sequenz gezeigt.

Der Begriff "Allel" ist einem Fachmann bekannt und betrifft allgemein eine beliebige Anzahl von alternativen Formen eines Gens oder Segmenten eines Chromosoms. Bevorzugt existieren im AB-Blutgruppensystem drei, die Blutgruppen der Katzen determinierende Allele: A, a^{ab} und b, wobei für die Vererbung gilt, dass Allel A dominant gegenüber a^{ab} und b und Allel a^{ab} dominant gegenüber b ist (A>a^{ab}>b). Es ist einem Fachmann folglich verständlich, dass eine heterozygote Katze mit nur einem A-Allel immer auch die serologische Blutgruppe A aufweist, wohingegen eine heterozygote Katze mit einem a^{ab}-Allel nur dann die Blutgruppe C aufweist, falls sie ebenfalls das b-Allel aufweist. Eine Katze der serologischen Blutgruppe B muss immer homozygot für das b-Allel sein.

Die Begriffe "homozygot" und "heterozygot" haben die Bedeutungen, die herkömmlicherweise mit diesen Begriffen in der Genetik assoziiert sind, und beziehen sich im Allgemeinen auf den Zustand, dass zwei identische (homozygote) oder nicht identische (heterozygote) Allele eines bestimmten Gens oder Segments des Chromosoms vorliegen.

Der Begriff "Mutation" betrifft eine genetische Veränderung der Nukleinsäure- und/oder Aminosäure-Sequenz. Bevorzugt ist die Mutation eine Substitution, eine Insertion, eine Deletion, eine Duplikation oder eine Inversion von mindestens einer Base in der Nukleinsäure-Sequenz oder dem Fragment davon, welches für eine *CMAH* codiert, sowie von mindestens einer Aminosäure in der Aminosäure-Sequenz.

Es ist einem Fachmann verständlich, dass die Mutation synonym sein kann, d.h. keinen Aminosäureaustausch in dem von der Nukleinsäure codierten Polypeptid zur Folge hat, oder nicht-synonym sein kann, d.h. einen Aminosäureaustausch und/oder einen Frame-Shift in dem von der Nukleinsäure codierten Polypeptid zur Folge hat. Es ist weiterhin verständlich, dass eine nicht-synonyme Mutation zu einem teilweisen oder vollständigen Funktionsverlust des von der Nukleinsäure codierten Polypeptids führen kann.

Es ist einem Fachmann weiterhin bekannt, dass, in Bezug auf Aminosäure-Sequenzen, einzelne Substitutionen, Deletionen oder Additionen an eine Nukleinsäure-, Peptid-, Polypeptid- oder Proteinsequenz, die eine einzelne Aminosäure oder einen kleinen Prozentsatz an Aminosäuren in der codierten Sequenz zur Folge haben, eine "konservativ modifizierte Variante" ist, bei der die Veränderung die Substitution einer Aminosäure durch eine chemisch ähnliche Aminosäure zur Folge hat. Konservative Substitutionstabellen, die funktionell ähnliche Aminosäuren bereitstellen, sind auf dem Fachgebiet gut bekannt. Solche konservativ modifizierten Varianten schließen zusätzlich polymorphe Varianten, Interspezies-Homologe und Allele der Erfindung nicht aus.

Es ist einem Fachmann weiterhin bekannt, dass der Begriff "Konservativ modifizierte Variante" für Aminosäure- und Nukleinsäure-Sequenzen gilt. In Bezug auf bestimmte Nukleinsäure-Sequenzen beziehen sich konservativ modifizierte Varianten auf diejenigen Nukleinsäuren, die für identische oder im Wesentlichen identische Aminosäure-Sequenzen codieren oder wenn die Nukleinsäure nicht für eine Aminosäure-Sequenz codiert, auf im Wesentlichen identische Sequenzen. Aufgrund der Degeneriertheit des genetischen Codes codiert eine große Anzahl von funktionell identischen Nukleinsäuren ein Protein. So ist eine "stille oder synonyme Variante" eine Veränderung in der Nukleotidsequenz, welche keine Veränderung des codierten Polypeptids bewirkt. Jede Nukleinsäure-Sequenz hierin, die für ein Polypeptid codiert, beschreibt auch jede mögliche stille, synonyme und/oder implizite Variation der Nukleinsäure.

Im Rahmen der Erfindung ist es erkannt worden, dass die drei Mutationen zur Bestimmung der Blutgruppe A oder Blutgruppe B der Katze eine G zu T Substitution an Position 179, eine T zu A Substitution an Position 268 und eine T-Deletion an Position 1322 sind und dass die vier Mutationen zur Bestimmung der Blutgruppe C der Katze eine G zu T Substitution an Position 179, eine T zu A Substitution an Position 268, eine C zu T Substitution an Position 364 und eine T-Deletion an Position 1322 sind. Weiter bevorzugt ist die Mutation ausgewählt ist aus der Gruppe umfassend eine Delta-53, das eine 18 Basenpaar Insertion-Deletion bei Position -53, das heißt im *CMAH* Exon 1b, betrifft, eine C zu T Substitution an Position 139, eine G zu A Substitution an Position 142, eine T zu C Substitution an Position 305, eine A zu C Substitution an Position 327, eine G zu A Substitution an Position 376, eine G zu T Substitution an Position 383, eine A zu C Substitution an Position 593, eine A zu C Substitution an Position 868, eine A zu G Substitution an Position 898, eine A-Deletion an Position 933 und eine G zu A Substitution an Position 1603. In Rahmen der Erfindung beginnt die Zählung bevorzugt beim Adenosin des Startcodons ATG, welches sich im Exon 1a befindet, wie in SEQ ID NO: 1 für die Nukleinsäure-Sequenz und in SEQ ID NO: 2 für die entsprechende Aminosäure-Sequenz gezeigt. Weiter bevorzugt werden 3, 4, 5, 6, 7, 8, 9, 10, 11, 12, 13, 14, 15, 16 oder mehr Mutation in dem Gen *CMAH* ermittelt. Erfindungsgemäß wird die Anwesenheit oder Abwesenheit der Mutationen an Positionen 1322, 179 und 268 in einem von beiden Allelen oder beiden Allelen des Gens *CMAH* bzw. die Anwesenheit oder Abwesenheit der Mutationen an Positionen 1322, 179, 268 und 364 in einem von beiden Allelen oder beiden Allelen des Gens *CMAH* ermittelt.

Es ist denkbar, dass zusätzlich die Anwesenheit von einer Mutation ausgewählt aus der Gruppe umfassend die Positionen Delta-53, 139, 142, 327, 364 und 1603 in einem von beiden Allelen und/oder die Abwesenheit von mindestens einer Mutation ausgewählt aus der Gruppe umfassend die Positionen Delta-53, 139, 142, 327, 364 und 1603 in beiden Allelen indikativ für die Blutgruppe A im AB-Blutgruppensystem ist. Bevorzugt ist die Anwesenheit einer Mutation heterozygot, das heißt, dass diese lediglich in einem Allel nachweisbar ist, und weiterhin bevorzugt ist die Abwesenheit von 2, 3, 4, 5, 6, 7 oder 8 der Mutationen, welche verständlicherweise nicht mit der einen heterozygot nachweisbaren, das heißt anwesenden, Mutation identisch sind, in beiden Allelen indikativ für die Blutgruppe A. Bevorzugt ist die mindestens eine Mutation ausgewählt aus der Gruppe umfassend die Positionen Delta-53, 139, 142, 327, 364 und 1603 wildtypisch homozygot, das heißt in keinem Allel nachweisbar und daher abwesend ist.

Weiterhin ist es denkbar, dass zusätzlich die Anwesenheit von mindestens einer Mutation ausgewählt aus der Gruppe umfassend die Positionen Delta-53, 139, 142 und 1603 in beiden Allelen und/oder die Anwesenheit von mindestens zwei Mutationen ausgewählt aus der Gruppe umfassend die Positionen Delta-53, 139, 142 und 1603 in einem von beiden Allelen indikativ für die Blutgruppe B im AB-Blutgruppensystem ist. Es ist einem Fachmann dabei verständlich, dass eine homozygot anwesende Mutation nicht gleichzeitig heterozygot anwesend sein kann und umgekehrt. Weiter bevorzugt ist die Anwesenheit von 2, 3, 4, 5, 6 oder 7 der Mutationen in einem von beiden Allelen oder in beiden Allelen indikativ für die Blutgruppe B. Noch mehr bevorzugt ist die Mutation homozygot, das heißt, dass diese in beiden Allelen nachweisbar und daher anwesend ist.

Zudem ist es denkbar, dass zusätzlich die Anwesenheit von einer Mutation an Position 327 und/oder von mindestens einer Mutation ausgewählt aus der Gruppe umfassend die Positionen 139, 142 und 1603 in einem von beiden Allelen oder in beiden Allelen indikativ für die Blutgruppe C im AB-Blutgruppensystem ist. Weiter bevorzugt ist die Anwesenheit von 3, 4, 5, 6, 7 oder 8 der Mutationen in einem oder in beiden Allelen indikativ für die Blutgruppe C. Bevorzugt ist die Mutation an der Position 327 homozygot, das heißt dass diese in beiden Allelen nachweisbar und daher anwesend ist, und die mindestens eine Mutation ausgewählt aus der Gruppe umfassend die Positionen 139, 142 und 1603 heterozygot, das heißt, dass diese in einem Allel nachweisbar ist.

Es ist einem Fachmann verständlich, dass die Begriffe "Nukleinsäure", "Gen", "cDNA", "mRNA", "Nukleotid", "Oligonukleotid" und "Polynukleotid" als austauschbare Synonyme für einander verwendet werden, um Desoxyribonukleotide oder Ribonukleotide und Polymere davon, entweder einzel- oder doppelsträngig, zu bezeichnen. Somit umfassen Nukleinsäuren beispielsweise, jedoch keinesfalls ausschließlich, bekannte Nukleotidanaloga oder modifizierte Rückstände, die synthetisch, natürlich vorkommend und nicht natürlich vorkommend, sind, welche ähnliche Bindungseigenschaften wie die Nukleinsäure aufweisen und die in ähnlicher Weise wie diese metabolisiert werden. Beispielsweise sind dies Phosphorthioate, Phosphoramidate, Methylphosphonate, chirale Methylphosphonate, 2-O-Methyl-Ribonukleotide, Peptid-Nukleinsäuren (PNAs). Weiterhin umfasst sind auch eine konservativ modifizierte Variante davon und komplementäre Sequenzen, sowie die explizit angegebene Sequenz. Insbesondere können degenerierte Codon-Substitutionen durch Erzeugen von Sequenzen erzielt werden, bei denen die dritte Position eines oder mehrerer ausgewählter (oder aller) Codons mit gemischten Basen und/oder Deoxyinosin-Resten substituiert ist.

Es ist einem Fachmann weiterhin verständlich, dass die Begriffe "Aminosäure", "Polypeptid", "Peptid" und "Protein" als austauschbare Synonyme für einander verwendet werden, um ein Polymer von Aminosäureresten zu beziehen. Die Begriffe gelten für Aminosäurepolymere, bei denen ein oder mehrere Aminosäurereste eine künstliche chemische Mimetika einer entsprechenden natürlich vorkommenden Aminosäure sind, sowie natürlich vorkommende Aminosäurepolymere und nicht natürlich vorkommende Aminosäurepolymere. Somit umfassen Aminosäuren beispielsweise, jedoch keinesfalls ausschließlich, natürlich vorkommende und synthetische Aminosäuren, sowie Aminosäureanaloga und Aminosäuremimetika, die auf ähnliche Weise wie die natürlich vorkommenden Aminosäuren funktionieren. Natürlich vorkommende Aminosäuren sind jene, die durch den genetischen Code codiert werden, sowie jene Aminosäuren, die später modifiziert werden. Aminosäureanaloga beziehen sich auf Verbindungen, die dieselbe chemische Grundstruktur wie eine natürlich vorkommende Aminosäure aufweisen, wie beispielsweise Homoserin, Norleucin, Methioninsulfoxid, Methioninmethylsulfonium. Solche Analoga haben modifizierte R-Gruppen (z. B. Norleucin) oder modifizierte Peptid-Grundgerüste, behalten jedoch die gleiche chemische Grundstruktur wie eine natürlich vorkommende Aminosäure bei. Aminosäuremimetika beziehen sich auf chemische Verbindungen, die eine Struktur aufweisen, die sich von der allgemeinen chemischen Struktur einer Aminosäure unterscheidet, die jedoch ähnlich wie eine natürlich vorkommende Aminosäure funktioniert.

Der Begriff "Haplotyp" betrifft einen Satz von eng verknüpften genetischen Markern, die auf einem Chromosom vorhanden sind und dazu neigen, zusammen vererbt zu werden (das heißt beispielsweise durch Rekombination nicht leicht zu trennen sind). Ein Haplotyp kann bevorzugt durch eine Reihe von Einzelnukleotid-Polymorphismen (SNPs) und/oder Mutationen, wie an anderer Stelle beschrieben, charakterisiert werden, die auf einem Chromosom vorhanden sind und die zusammen vererbt werden.

Im Rahmen der Erfindung ist es des Weiteren erkannt worden, dass die Blutgruppe der Katze im AB-Blutgruppensystem basierend auf der Anwesenheit oder Abwesenheit von mindestens drei Mutationen in einem von beiden Allelen oder in beiden Allelen bestimmt wird, wobei die Anwesenheit oder Abwesenheit indikativ für die Blutgruppe A, B oder C ist. Dabei ist es erkannt worden, dass die Anwesenheit von einer der Mutationen an Positionen 1322, 179 und 268 in einem von beiden Allelen und/oder die Abwesenheit von mindestens einer der Mutationen in beiden Allelen indikativ für die Blutgruppe A ist, sowie, dass die Anwesenheit von mindestens einer der genannten Mutationen in beiden Allelen und/oder die Anwesenheit von mindestens zwei der Mutationen in einem von beiden Allelen indikativ für die Blutgruppe B ist. Weiter ist es erkannt worden, dass die Anwesenheit von der Mutation an Position 364 und/oder von mindestens einer der Mutationen ausgewählt aus der Gruppe umfassend die Positionen 179, 268 und 1322 in einem von beiden Allelen oder in beiden Allelen indikativ für die Blutgruppe C ist.

Es ist einem Fachmann dabei bekannt, wie die Anwesenheit oder Abwesenheit mindestens einer Mutation in einer biologischen Probe mit aus dem Stand der Technik bekannten Techniken bestimmt werden kann.

Der Begriff "Blutgruppe" betrifft im Allgemeinen eine von vielen Gruppen/Typen, in die das Blut eines Individuums auf der Grundlage der Anwesenheit oder Abwesenheit von spezifischen Antigenen, produziert durch das gleiche Gen, auf der Oberfläche von Blutzellen kategorisiert werden kann. Im Rahmen der Erfindung betrifft der Begriff "Blutgruppe" dabei bevorzugt das AB-Blutgruppensystem, wobei bei der Katze drei Blutgruppen im AB-Blutgruppensystem, nämlich A, B und C (AB), bekannt sind.

Der Begriff "biologische Probe" betrifft ein Material einer Katze, welche eine Nukleinsäure enthält, die für ein mutiertes CMAH-Polypeptid codiert. Derartige Proben sind einem Fachmann bekannt, wie beispielsweise zell- und kernhaltige (DNA- und/oder RNA-enthaltene) Proben.

Der Begriff "Verfahren zur genotypischen Bestimmung der Blutgruppe" betrifft ein Verfahren zur genotypischen Bestimmung der Blutgruppe im AB-Blutgruppensystem bzw. zur Unterscheidung zwischen den Blutgruppen A, B oder C einer Katze. Das erfindungsgemäße Verfahren kann zusätzliche Schritte enthalten, welche nach oder zwischen den explizit aufgeführten essentiellen Schritten a) bis b) liegen. Bevorzugt ist das Verfahren teilweise oder vollständig automatisierbar.

Der Begriff "genotypische Bestimmung der Blutgruppe" der Katze betrifft die Ermittlung der Blutgruppe im AB-Blutgruppensystem bzw. die Unterscheidung zwischen den für Katzen bekannten Blutgruppen A, B oder C basierend auf der Anwesenheit oder Abwesenheit von mindestens drei Mutation in einem von beiden Allelen oder in beiden Allelen des felinen Gens *CMAH* in der biologischen Probe derselben. Bevorzugt wird dabei der Genotyp des CMAH-Gens bestimmt, welcher mit der Ausprägung der Blutgruppe A, B oder C im AB-Blutgruppensystem korreliert. Es ist für einen Fachmann verständlich, dass die Bestimmung bevorzugt semiquantitativ, quantitativ, direkt und/oder indirekt mit aus dem Stand der Technik bekannten Mitteln, wie beispielsweise einem Algorithmus, erfolgt.

Es ist einem Fachmann verständlich, dass eine genotypische Bestimmung in der Regel nicht zu 100 Prozent korrekt sein kann. Der Begriff betrifft daher eine statistisch signifikante Wahrscheinlichkeit, was die Genauigkeit der genotypischen Bestimmung der Blutgruppe im AB-Blutgruppensystem betrifft. Ob eine derartige Bestimmung statistisch signifikant ist, kann ohne erfinderisch tätig zu werden von einem Fachmann mittels in der Fachwelt bekannter Verfahren bestimmt werden. Beispielsweise sind statistische Evaluierungstools zu nennen, wie beispielsweise die Bestimmung des Konfidenzintervalls, des p-Wertes, des Student's-t-Tests, der Mann-Whitney-Bestimmung usw.. Die entsprechenden Intervalle sind mindestens 90 %, mindestens 95 %, mindestens 97 %, mindestens 98 % oder mindestens 99 % korrekt. Die p-Werte sind bevorzugt 0,1, 0,05, 0,01, 0,005 oder 0,0001. Bevorzugt ist diese Bestimmung der Blutgruppe der Katze im Rahmen der vorliegenden Erfindung mindestens 60 %, mindestens 70 %, mindestens 80 %, mindestens 90 %, mindestens 95 %, mindestens 96 %, mindestens 97 %, mindestens 98 % oder mindestens 99 % korrekt.

Mittels der erfindungsgemäßen Verfahren ist es somit möglich, die Genauigkeit der genotypischen Bestimmung der Blutgruppe der Katze signifikant zu erhöhen, sowie zuverlässig zwischen den drei für Katzen bekannten Blutgruppen im AB-Blutgruppensystem zu unterscheiden. Auf diese Weise ist es möglich, schnell und mit nur einer Untersuchung des Gens *CMAH* der betreffenden Katze eine zuverlässige und hinreichend genaue genotypische Bestimmung der Blutgruppe zu erhalten, so dass zusätzliche Kosten für etwaige Wiederholungen oder lange laboranalytische Verfahren vollständig entfallen. Dies bedeutet zudem, dass das Verfahren für die Katze nicht nur sehr schonend ist, zudem bleibt der Katze eine Wiederholung der Probenentnahme erspart.

Vorteilhafte Weiterbildungen der Erfindung, welche einzeln oder in Kombination realisierbar sind, sind in den Unteransprüchen dargestellt.

In einer Weiterbildung der Erfindung ist es denkbar, dass das Ermitteln umfasst:
a) Amplifizieren einer Nukleinsäure, wobei die Nukleinsäure ausgewählt ist aus der Gruppe umfassend
   i) eine Nukleinsäure-Sequenz wie in SEQ ID NO: 1 gezeigt; und
   ii) eine Nukleinsäure-Sequenz, welche ein Polypeptid codiert, das eine Aminosäure-Sequenz wie in SEQ ID NO: 2 gezeigt aufweist; und
   iii) eine Nukleinsäure-Sequenz, welche ein Polypeptid codiert, das mindestens 50 % identisch zu einem Polypeptid ist, das von den Nukleinsäure-Sequenzen aus i) oder ii) codiert wird, wobei das Polypeptid die feline CMAH ist; und
   iv) eine Nukleinsäure-Sequenz für ein Fragment einer Nukleinsäure aus i), ii) oder iii), wobei das Fragment ein Polypeptid codiert, wobei das Polypeptid die feline CMAH ist; und
b) Detektieren der amplifizierten Nukleinsäure-Sequenz.

Der Begriff "Amplifizieren" betrifft im Allgemeinen eine biochemische, chemische und/oder enzymatische Reaktion, mittels welcher eine Erhöhung der Kopien einer Nukleinsäure-Sequenz aus der biologischen Probe erreicht wird. Bevorzugt ist es denkbar, dass die Nukleinsäure-Sequenz aus der biologischen Probe teilweise oder vollständig isoliert ist. Weiter bevorzugt ist es denkbar, dass die Nukleinsäure-Sequenz nicht aus der biologischen Probe isoliert ist, das heißt, dass die Nukleinsäure-Sequenz aus der biologischen Probe amplifizierbar ist. Einem Fachmann sind aus dem Stand der Technik derartige Amplifikationsreaktionen bekannt, wie beispielsweise, jedoch keinesfalls ausschließlich, Polymerase-Kettenreaktion (PCR), Ligase-Kettenreaktion (LCR), Multi-displacement Amplification (MDA), Transkriptions-vermittelte Amplifikation (TMA), Nukleinsäure-Sequenz-basierte Amplifikation (NASBA), Rolling-Circle-Amplifikation (RCA) und branched-DNA-Assay (bDNA). Es sind einem Fachmann auch die entsprechenden Komponenten der genannten Techniken, wie beispielsweise Primer, bekannt.

Der Begriff "Primer" betrifft eine Nukleinsäure-Sequenz, welche die Synthese einer isolierten Nukleinsäure-Sequenz in einer entsprechenden Amplifikationsreaktion prägt. Typischerweise umfasst ein Primer weniger als etwa 100 Nukleotide und umfasst vorzugsweise weniger als etwa 30 Nukleotide. Beispielhafte Primer liegen im Bereich von etwa 5 bis etwa 25 Nukleotiden.

Der Begriff "isolierte Nukleinsäure" betrifft eine Nukleinsäure oder ein Polynukleotid, welche im Wesentlichen oder hauptsächlich frei von Komponenten ist, welche diese normalerweise begleiten bzw. wie es in seinem nativen Zustand vorkommt. Bevorzugt wird die Nukleinsäure oder das Polynukleotid einerseits aus der biologischen Probe der Katze isoliert. Weiter bevorzugt wird dies andererseits, unter Verwendung aus dem Stand der Technik bekannter Techniken, wie beispielsweise, jedoch keinesfalls ausschließlich Polyacrylamid-Gelelektrophorese oder Hochleistungsflüssigkeitschromatographie erreicht. So ist es beispielsweise denkbar, dass eine isolierte Nukleinsäure vom einem regulatorischen Bereich und/oder open readings frames getrennt wird, welche diese, bevorzugt das *CMAH-*Gen*,* flankieren und/oder andere Proteine als CMAH codieren. Der Begriff "isoliert" bedeutet zudem, dass eine Nukleinsäure oder ein Protein im Wesentlichen eine Bande in einem elektrophoretischen Gel erzeugt. Insbesondere bedeutet dies, dass die Nukleinsäure oder das Protein zu mindestens 85 %, besonders bevorzugt zu mindestens 95 % und am meisten bevorzugt zu mindestens 99 % rein ist.

Im Rahmen der Erfindung sind dabei Nukleinsäuren umfasst, welche sich auf polymorphe Varianten, Fragmente, Allele, Mutanten und Interspezies-Homologe der *CMAH,* wie in SEQ ID NO:1 und/oder SEQ ID NO: 2 gezeigt, beziehen. Es ist einem Fachmann verständlich, dass die Nukleinsäuren natürlich vorkommende oder rekombinante Moleküle umfassen. Die Nukleinsäuren haben bevorzugt i) eine Nukleinsäure-Sequenz wie in SEQ ID NO: 1 gezeigt. Weiter bevorzugt weise diese eine Nukleinsäure-Sequenz auf, die eine Identität von mindestens 50 %, mindestens 60 %, mindestens 65 %, mindestens 70 %, mindestens 75 %, mindestens 80 %, mindestens 85 %, mindestens 90 %, mindestens 95 %, mindestens 96 %, mindestens 97 %, mindestens 98 %, mindestens 99 % oder mehr, vorzugsweise über einen Bereich von mindestens etwa 25, 100, 200, 500, 1000 oder mehr aufeinanderfolgenden Nukleotiden aufweisen. Alternativ bevorzugt haben diese ii) eine Nukleinsäure-Sequenz, welche ein Polypeptid codiert, das eine Aminosäure-Sequenz wie in SEQ ID NO: 2 gezeigt aufweist. Weiter alternativ bevorzugt haben diese iii) eine Nukleinsäure-Sequenz, welche ein Polypeptid codiert, das mindestens 50 %, mindestens 60 %, mindestens 65 %, mindestens 70 %, mindestens 75 %, mindestens 80 %, mindestens 85 %, mindestens 90 %, mindestens 95 %, mindestens 96 %, mindestens 97 %, mindestens 98 %, mindestens 99 % oder mehr Identität, vorzugsweise über einem Bereich von mindestens etwa 25, 50, 100, 200, 500, 1000 oder mehr aufeinanderfolgenden Aminosäuren zu einem Polypeptid aufzeigt, das von den Nukleinsäure-Sequenzen aus i) oder ii) codiert wird, wobei das Polypeptid die feline CMAH ist, wie in SEQ ID NO: 1 und in SEQ ID NO: 2 gezeigt.

Einem Fachmann sind für einen Vergleich von Sequenzen, beispielsweise einer isolierten Nukleinsäure-Sequenz mit der Nukleinsäure-Sequenz wie in SEQ ID NO: 1 gezeigt und/oder einer isolierten Nukleinsäure-Sequenz mit der Aminosäure-Sequenz wie in SEQ ID NO: 2 gezeigt eine Vielzahl von aus dem Stand der Technik bekannte Techniken bekannt.

Der Begriff "Vergleich" betrifft den teilweisen oder vollständigen Vergleich der Nukleinsäuren miteinander, mit den entsprechend codierten Aminosäuren und/oder mit einer aus dem Stand der Technik bekannten Referenz-Sequenz. Dabei versteht es sich, dass ein Vergleich, wie er hier angewendet wird, sich auf einen Vergleich entsprechender Parameter bezieht. Im Rahmen der Erfindung kann der Vergleich manuell und/oder computerunterstützt durchgeführt werden. Für einen computergestützten Vergleich sind alle einem Fachmann bekannten Mittel denkbar, wie beispielsweise ein Computer und/oder ein Computerprogramm, wie beispielsweise die BLAST- und BLAST 2.0-Algorithmen. Ein Computerprogramm kann zusätzlich das Ergebnis des Vergleichs evaluieren, beispielsweise automatisch eine Beurteilung der entsprechend von der Nukleinsäure codierten Aminosäure, sowie eine Vorhersage der zu erwartenden Primär-, Sekundär-, Tertiär- und/oder Quartärstruktur, sowie eine Vorhersage zu Bereichen, wie aktiven oder inaktiven Bereichen, des Polypeptids treffen.

Der Begriff "Detektieren" betrifft im Allgemeinen die Analyse und/oder den Nachweis der Nukleinsäure-Sequenz, insbesondere der Anwesenheit oder Abwesenheit von mindestens einer Mutation in der Nukleinsäure-Sequenz. Einem Fachmann sind aus dem Stand der Technik eine Vielzahl an Detektionstechniken zur Evaluierung von Nukleinsäuren auf das Vorhandensein einer einzelnen Basenveränderung bekannt, wie beispielsweise eine oben genannte Amplifikationsreaktion, eine Sonde und/oder eine Sequenzierung. Auf diese Weise wird es vorteilhafterweise erreicht, dass die Bestimmung der Blutgruppe der Katze im AB-Blutgruppensystem zuverlässig, sicher, schnell und einfach realisierbar ist.

In noch einer Weiterbildung der Erfindung ist es denkbar, dass die Katze eine Großkatze, eine Kleinkatze, wie beispielsweise eine Wildkatze, eine Hauskatze, eine Rassekatze und/oder ein Mischling ist. Bevorzugt ist die Katze beispielsweise, jedoch keinesfalls ausschließlich eine Katze ausgewählt aus der Gruppe umfassend die Familie Felidae, die Unterfamilie Felinae, Pantherinae und Acinonychinae, die Gattung Altwelt-Wildkatze, Caracal, Asiatische Goldkatzen, Acinonyx, Echte Katzen, Pumas, Pardelkatzen, Luchse, Otocolobus, Leptailurus, Altkatzen, Neofelis, Eigentliche Großkatzen und Pardofelis, die Arten Hauskatze, Kanaani-Katze, Gepard, Luchs, Ozelot, Löwe, Tiger, Jaguar, Leopard, Schneeleopard, Nebelparder, Sunda-Nebelparder, Karakal, Graukatze, Rohrkatze, Sandkatze, Schwarzfußkatze, Wildkatze, Afrikanische Goldkatze, Marmorkatze, Borneo-Goldkatze, Asiatische Goldkatze, Pampaskatze, Kleinfleckkatze, Chilenische Waldkatze, Andenkatze, Tigerkatze, Langschwanzkatze, Serval, Kanadischer Luchs, Eurasischer Luchs, Pardelluchs, Rotluchs, Manul, Bengalkatze, Flachkopfkatze, Rostkatze, Fischkatze, Puma, Jaguarundi, Rassekatze und/oder ein Mischling davon. Im Rahmen der Erfindung ist es dabei weiter bevorzugt, dass die Rassekatze beispielsweise, jedoch keinesfalls ausschließlich eine Rassekatze ausgewählt aus der Gruppe umfassend Ragdoll, Britisch Kurzhaar, Britisch Langhaar, Sibirische Katze und die nah verwandte Point-Variante Neva Masquarade, Maine-Coon, Heilige Birma, Schottische Faltohrkatze, Türkisch Angora, Perser und Abessinier ist.

Es ist weiterhin denkbar, dass die biologische Probe eine Körperflüssigkeit, wie beispielsweise Vollblut, Serum, Plasma, Liquor, Urin, Lymphflüssigkeit, verschiedene äußere Sekrete der Atemwege, des Darms und des Urogenitaltrakts, Tränen, Speichel, weiße Blutkörperchen, Myelome und dergleichen, eine biologische Flüssigkeiten, wie beispielsweise ein Zellextrakt und/oder ein Zellkulturüberstand, ein Gewebe und/oder eine Zellprobe, wie eine Haut- und/oder Fellprobe, ein Gewebeschnitt, wie beispielsweise eine Biopsie- und/oder Autopsieprobe, und/oder ein Gefrierschnitt ist. Diese Proben sind auf dem Fachgebiet gut bekannt.

Es wird davon ausgegangen, dass die Definitionen und/oder die Ausführungen der oben genannten Begriffe für alle in dieser Beschreibung im Folgenden beschriebenen Aspekte gelten, sofern nichts anderes angegeben ist.

Es ist einem Fachmann auch bekannt, dass sich der Begriff "Antikörper" auf ein Polypeptid bezieht, welches eine Gerüstregion von einem Immunglobulin-Gen oder Fragmenten davon umfasst, die spezifisch ein Antigen bindet und erkennt. Die erkannten Immunglobulin-Gene umfassen die Gene der konstanten Region von Kappa, Lambda, Alpha, Gamma, Delta, Epsilon und Mu sowie die Gene der variablen Region des Immunglobulins. Leichte Ketten werden entweder als Kappa oder Lambda klassifiziert. Schwere Ketten werden als gamma, mu, alpha, delta oder epsilon klassifiziert, die ihrerseits die Immunglobulinklassen IgG, IgM, IgA, IgD bzw. IgE definieren. Der Antikörper ist bevorzugt ein monoklonaler, ein polyklonaler oder ein chimärer Antikörper. Noch mehr bevorzugt ist der Antikörper gegen ein Immunogen gerichtet, das spezifisch ein Polypeptid bindet, welches von einem *CMAH-*Gen (wie in SEQ ID NO: 1 gezeigt), einer cDNA oder einem Fragment davon codiert wird, einschließlich von einem mutierten *CMAH*-Gen, cDNA oder einem Fragment davon codierten Polypeptiden (wie in SEQ ID NO: 2 gezeigt).

Weitere Einzelheiten, Merkmale und Vorteile der Erfindung ergeben sich aus der nachfolgenden Beschreibung der bevorzugten Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in der Figur schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigt:

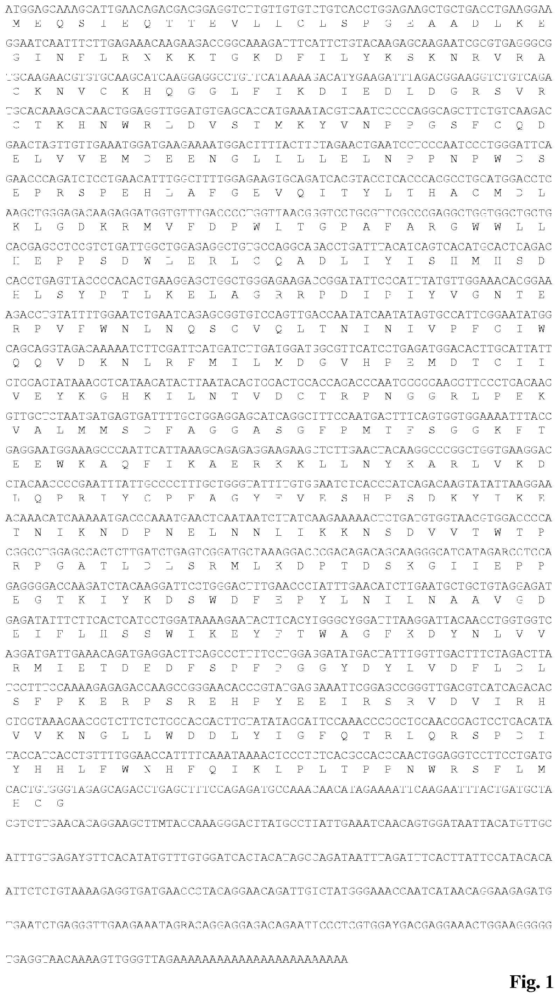

- **Fig.1**: eine Gegenüberstellung der Nukleinsäure-Sequenz des felinen *CMAH,* wie in SEQ ID NO: 1 gezeigt, mit der entsprechend codierten Aminosäure-Sequenz, wie in SEQ ID NO: 2 gezeigt.

In Fig. 1 sind die sich entsprechenden Sequenzen (SEQ ID NO: 1 und 2) gegenübergestellt, welche im Rahmen der vorliegenden Erfindung für die Identifizierung der SNPs und Mutationen, beziehungsweise deren Benennung, verwendet wurden. Diese beginnen bei der Base Adenin des Startcodons, welches in Exon 1a des *CMAH* lokalisiert ist, da dies ebenfalls der Startpunkt zur Identifizierung im aktuellen Stand der Technik ist und somit ein Vergleich der SNPs und Mutationen einfacher vonstattengeht.

Die folgenden Ausführungsbeispiele dienen lediglich dazu, die Erfindung zu illustrieren. Sie sollen den Gegenstand der Patentansprüche in keiner Weise beschränken.

### Beispiele:

### Beispiel 1: DNA/RNA-Isolierung aus dem Blut von Katzen

Die Isolierung der DNA der Probandentiere wird vollautomatisch von dem MagNA Pure 96 (Roche Molecular Systems) mittels des MagNA Pure 96 DNA and Viral NA Small Volume- Kits (Roche) durchgeführt. Hierfür werden 200 µl des jeweiligen Probengemischs (meistens EDTA-Blut) in die dafür vorgesehene Schiene pipettiert. Man erhält dadurch etwa 100 µl DNA/RNA-Lösung.

### Beispiel 2: Sequenzierung

Die Suche nach neuen genetischen Varianten, die eine Assoziation mit der Differenzierung der Blutgruppen aufweisen könnten, erfordert die Sequenzierung aller 16 Exons des *CMAH-*Gens*.* Vor der Sequenzierung muss eine Amplifikation dieser Exons mithilfe einer PCR durchgeführt werden. Aufgrund der relativ langen Introns zwischen den Exons sind, mit Ausnahme der Exons 5 und 6, für jedes Exon eigene Primerpaare nötig. Die nachfolgend in Tabelle 1 dargestellten Oligonukleotide werden als Primer für die Untersuchung der biologischen Probe verwendet.

**Tabelle 1: verwendete Oligonukleotide**

| **Primer** | **(5'UTR → 3'UTR) Sequenz** | **SEQ ID NO:** |
|---|---|---|
| Exon 1 Forward | TGT AAA ACG ACG GCC AGT TGA GCA AGC AGA GCG TGC AT | 3 |
| Exon 1 Reverse | GCA GGA AAC AGC TAT GAC AAG TGA GCA CAC ACG AAG ATT ATG | 4 |
| Exon 2 Forward | TGT AAA ACG ACG GCC AGT TGA AGT GGT GCT CAA GCC CCT CAT TTA | 5 |
| Exon 2 Reverse | GCA GGA AAC AGC TAT GAC CTG GTC ACA GTC TTG TCC TAG GCA C | 6 |
| Exon 3 Forward | TGT AAA ACG ACG GCC AGT TGA CCC TTT GAT TTT TGG TAC TCT GTG TAC | 7 |
| Exon 3 Reverse | GCA GGA AAC AGC TAT GAC CTA ATA CAC ACA CAT AAA TGG ACA TCT | 8 |
| Exon 4 Forward | TGT AAA ACG ACG GCC AGT TGA TAA AAC CAA CCG GGA GAG TTT CTA T | 9 |
| Exon 4 Reverse | GCA GGA AAC AGC TAT GAC CAC ACA TGA AAA CTG TAT GAT ATT CGC | 10 |
| Exon 5-6 Forward | TGT AAA ACG ACG GCC AGT TGA GGA GCA AAC TGT CTC TTC TAC TAG GTG | 11 |
| Exon 5-6 Reverse | GCA GGA AAC AGC TAT GAC CGA AAT GAA GAT AGT AGA AGA TGG TTA | 12 |
| Exon 7 Forward | TGT AAA ACG ACG GCC AGT TGA TTC CTT GGA TAG AAG TAA TTC TTC TCT | 13 |
| Exon 7 Reverse | GCA GGA AAC AGC TAT GAC CGT TTC CCA GTT AAG ACT GTA AAC TTC | 14 |
| Exon 8 Forward | TGT AAA ACG ACG GCC AGT TGA AGA GAG CAC TTT CTA CTC ATG ACA CA | 15 |
| Exon 8 Reverse | GCA GGA AAC AGC TAT GAC CAG ACA ATC TCT CTG AAT GGA TTC TAG | 16 |
| Exon 9 Forward | TGT AAA ACG ACG GCC AGT TGA AAG ACA AAG TAT AAG TAC AAC TTC TTG | 17 |
| Exon 9 Reverse | GCA GGA AAC AGC TAT GACCAC AAA GAC TTA ATA TTC CTA CAA ACG | 18 |
| Exon 10 Forward | TGT AAA ACG ACG GCC AGT TGA CAA AAG ATG TGG AGT TTT CCC ATA GT | 19 |
| Exon 10 Reverse | GCA GGA AAC AGC TAT GAC CAA CAA TCG TGG GAC AAG AAT GCT | 20 |
| Exon 11 Forward | TGT AAA ACG ACG GCC AGT TGA GAG GGA CTA TAC CGA ACT CCA ATT CAT T | 21 |
| Exon 11 Reverse | GCA GGA AAC AGC TAT GAC CGA CAA CTA GCA CAG TAA AGG AAG GTG | 22 |
| Exon 12 Forward | TGT AAA ACG ACG GCC AGT TGA TGC GTT CTG TAG ATG AGG AGA CTG C | 23 |
| Exon 12 Reverse | GCA GGA AAC AGC TAT GAC CGA ATG AAG CTC TAA TGG TGG AAT CT | 24 |
| Exon 13 Forward | TGT AAA ACG ACG GCC AGT TGA AAA TGC CAG GCA CTG AAA CAG TA | 25 |
| Exon 13 Reverse | GCA GGA AAC AGC TAT GAC CGA TTT AGC TCT GAA CAC TCA CTG CC | 26 |
| Exon 14/15 3'-UTR Forward | TGT AAA ACG ACG GCC AGT TGA CGT ATA GTT CAG | 27 |
| Exon 14/15 3'-UTR Reverse | GCA GGA AAC AGC TAT GAC CCC TGA CTC TCT | 28 |
| Exon 1a Forward | TGT AAA ACG ACG GCC AGT TGA AAT TTA CAG | 29 |
| Exon 1a Reverse | GCA GGA AAC AGC TAT GAC CTA GCA AAC ATA | 30 |
| 5'-UTR Forward | TGT AAA ACG ACG GCC AGT TGA CGC TCT CCT TGC TCA GTG TTG | 31 |
| 5'-UTR Reverse | GCA GGA AAC AGC TAT GAC CCA ACA CTG AGC | 32 |
| Forward-Primer T268A-BG5F (Genotyping-Assay 268) | CAC AAA GCA CAA CTG GAG GTT | 33 |
| Reverse-Primer T268A-BG5R (Genotyping-Assay 268) | CAA CTA GTT CGT CTT GAC AGA AGC T | 34 |
| Reporter-Sonde T268A-BG5V1 (VIC-markiert) | CAC CAT GAA ATA CGT CAA T | 35 |
| Reporter-Sonde T268A-BG5M1 (FAM-markiert) | ACC ATG AAA AAC GTC AAT | 36 |
| Forward-Primer G142A-BG4F (Genotyping-Assay) | GCA AAG ATT TCA TTC TGT ACA AGA GCA A | 37 |
| Reverse-Primer G142A-BG4R (Genotyping-Assay) | CTT GAT GCT TGC ACA CGT TCT T | 38 |
| Reporter-Sonde G142A-BG4V2 (VIC) | CCC TCA CGC GAT TC | 39 |
| Reporter-Sonde G142A-BG4M2 (FAM) | CCC TCA TGC GAT TC | 40 |
| Forward-Primer CMAH_F (Genotyping-Assay) | GGT GCA ACG GAA TCA GTA GTG A | 41 |
| Reverse-Primer CMAH_R (Genotyping-Assay) | GCC TCT CCC TGG GAA TTC TG | 42 |
| Reporter-Sonde CMAH_wt (YAK-BBQ) | YAK-CAG CTT CGG TTG CTC GTT TGC TC-BBQ | 43 |
| Reporter-Sonde CMAH_mut (FAM-BBQ) | FAM-ACG GTG GTT GCT CGT TTG CTC C-BBQ | 44 |

Die PCR wird mit dem FastStart PCR-Master-Kit (Roche) nach Herstellerangaben durchgeführt, welches bereits einen geeigneten Reaktionspuffer, eine Taq-Polymerase und die nötigen dNTPs enthält. Pro Exon wird ein 50 µl-Ansatz nach Herstellerangaben zusammengestellt. Basierend auf einer vorausgegangenen Gradienten-PCR wurde ein Thermocycler-Programm zur Amplifikation der 16 Exons bei standardisierten Ampifikationstemperaturen unter Standard-Bedingungen durchgeführt.

Das PCR-Produkt wird mittels einer Gelelektrophorese nachgewiesen. Hierfür werden 8 µl des Ansatzes in die Taschen eines 4 %-igen Agarose-Gels mit Ethidium-Bromid pipettiert (Invitrogen, Thermo Fisher Scientific), die mit 12 µl H₂O befüllt sind. Das Gel wird 20 Minuten in dem Elektrophorese-Gerät E-Base (Invitrogen) unter Spannung gesetzt und anschließend unter UV-Licht fotografiert. Jeweils 15 µl des PCR-Produktes werden sequenziert oder wie folgt weiterbehandelt.

Das PCR-Produkt muss zuerst aufgereinigt werden. Hierfür wird das MinElute PCR-Purification Kit (250) von Qiagen verwendet. Auf die gekühlten Säulen wird das aufzureinigende PCR-Produkt gegeben, das zuvor mit 250 µl Binding Buffer vermischt wurde. Die Säule wird bei 13.000 rpm für eine Minute zentrifugiert und der Durchlauf verworfen. Es werden 600 µl Washing Buffer auf die Säule gegeben und der Zentrifugationsschritt wiederholt. Nach dem Verwerfen des Durchflusses wird ein Trockenzentrifugationsschritt durchgeführt. Die Säule wird anschließend in ein frisches Reaktionsgefäß gestellt und mit 30 µl Elution-Buffer befüllt. Nach einer Minute Zentrifugation bei 8.000 rpm befindet sich das aufgereinigte PCR-Produkt im Durchfluss der Säule.

Das aufgereinigte PCR-Produkt wird als Template für eine Standard-PCR verwendet. Hierbei wird das BigDye Terminator v1.1 Cycle Sequencing Kit von Roche nach Herstellerangaben verwendet. Dieses Gemisch wird laut Hersteller mit einem Thermocycler-Programm bei standardisierten Ampifikationstemperaturen unter Standard-Bedingungen behandelt.

Das PCR-Produkt wird nun 1:1 mit Wasser verdünnt und die Spin-Columns des DyeEx 2.0 Spin Kits (250) von Qiagen vorbereitet, indem sie 5 Minuten bei 2800 rpm trockenzentrifugiert und auf frische Reaktionsgefäße gestellt werden. Die verdünnte Probe wird nun auf die Säule gegeben und 5 Minuten bei 2800 rpm zentrifugiert. Der Durchfluss wird die gewünschten Proben erhalten, die in den Genetic Analyzer zur Kapillarelektrophorese gegeben werden.

### Beispiel 3: SNP-Genotyping-Assay

Das Genotyping-Assay basiert auf zwei verschiedenen Reportersonden pro untersuchtem SNP, die mit jeweils einem Fluorophor und einem Quencher hybridisiert sind. Die Sonde besteht aus einem Oligonukleotid, das komplementär zu einem Bereich um den zu untersuchenden SNP ist. Jeweils eine der Sonden bindet spezifisch an das wildtypische oder das mutierte Allel, das zwischen den beiden Primern zur Amplifikation lokalisiert ist. Ist die Sonde intakt, so unterdrückt der Quencher die Fluoreszenz des Farbstoffes durch die relative Nähe der beiden Stoffe. Durch die Exonukleaseaktivität der DNA-Polymerase wird das Fluorophor von der Sonde abgetrennt, wenn diese am Template-Strang gebunden hat. Liegt das Fluorophor nun getrennt vom Quencher vor, so wird die Fluoreszenz nicht mehr von dem Quencher unterdrückt und kann detektiert werden.

Für das Assay wird das Fast Start Essential MM-Kit (Roche) nach Herstellerangaben verwendet. Die Primer für die Amplifikation werden mit einer Endkonzentration von 0,02 pM und die Sonden mit einer Endkonzentration von 0,6 pM in einem Primermix angesetzt. Bei dem PCR-Ansatz wird für die SNP-Assays von 142 und 268 und für Delta-53 ein unterschiedlicher *modus operandi* gewählt. Die Ansätze werden im Light Cycler (Roche) in einem Standard-Programm inkubiert und auf Fluoreszenz analysiert.

Die Fluoreszenz wird auf den beiden Fluoreszenzkanälen der Fluorophoren VIC (Absorption 538, Emission 554) und FAM (Absorption 494, Emission 518) gemessen und gegeneinander aufgetragen. In den durchgeführten Untersuchungen haben sich erwartungsgemäß drei distinkte Gruppen ergeben. Eine der Gruppen zeigt nur das Signal der wildtypischen Sonde (VIC), die andere nur das der mutierten Sonde (FAM) und die dritte Gruppe weist beide Signale auf.

### Beispiel 4: Sequenzanalyse bei Katzen der Blutgruppe B in verschiedenen Rassekatzen

Das Gen *CMAH* setzt sich aus 16 Exons zusammen, wobei zwei Isoformen des Enzyms existieren, die beide notwendig sind, um die katalytische Aktivität zu gewährleisten. Die Isoformen *CMAH* 1a und *CMAH* 1b entstehen durch unterschiedliches Splicing der Leader-Exons. Bei *CMAH* 1a wird das Exon 1a an das Exon 2 gespliced, bei *CMAH* 1b dementsprechend das Exon 1b. Die im Rahmen der Erfindung durchgeführte Sequenzierung umfasst die beschriebenen 16 Exons, nämlich die Exons 2 bis 15 und die beiden möglichen Starter-Exons 1a und 1b. Letztere enthalten die 5'-UTR der mRNA und das Exon 15 bildet das 3'-UTR. Die Identifizierung der SNPs und Mutationen, beziehungsweise deren Benennung beginnt bei der Base Adenin des Startcodons, welches in Exon 1a lokalisiert ist, da dies ebenfalls der Startpunkt zur Identifizierung im aktuellen Stand der Technik ist und somit ein Vergleich der SNPs einfacher vonstattengeht.

Die Sequenzierungsergebnisse der 71 Testexemplare zeigen, zusätzlich zu den 19 bereits aus dem Stand der Technik bekannten Varianten des felinen CMAH-Gens, 17 neue Mutationen innerhalb der Exons des Gens auf. Bei acht dieser Mutationen (103G>A, 141C>T, 213A>G, 501G>A, 636G>A, 1392T>C, 1452T>C und 1649G>A) handelt es sich um synonyme Mutationen, die keinen Aminosäureaustausch zur Folge haben. Zwei weitere sind als Deletionen zu klassifizieren (933delA und 1322delT), die beide das Gen um eine Base verkürzen und einen Frame-shift der nachfolgenden Codons nach sich ziehen. Die restlichen sieben neuen Mutationen (305T>C, 376G>A, 383G>T, 593A>C, 868A>C und 898A>G) sind nicht-synonyme Mutationen, die folglich einen Aminosäureaustausch mit sich bringen (siehe Tabelle 2).

**Tabelle 2: Neu gefundene Mutationen im CMAH-Gen von Rassekatzen**

| **Stelle der Punktmutation** | **Auswirkung auf die Aminosäure-Sequenz** |
|---|---|
| 103G>A | Keine |
| 141C>T | Keine |
| 213A>G | Keine |
| 501G>A | Keine |
| 636G>A | Keine |
| 1392T>C | Keine |
| 1452T>C | Keine |
| 1649G>A | Keine |
| 933delA | Frame-Shift |
| 1322delT | Frame-Shift |
| 305T>C | Lysin > Prolin |
| 376G>A | Serin > Leucin |
| 383G>T | Arginin > Isoleucin |
| 593A>C | Histidin > Prolin |
| 868A>C | Threonin > Prolin |
| 898A>G | Lysin > Glutaminsäure |

### Beispiel 5: Vollständig sequenzierte Katzen geordnet nach Rassen und Blutgruppen

Im Rahmen der vorliegenden Erfindung konnte das CMAH-Gen von insgesamt 71 Katzen erfolgreich sequenziert werden. Wie aus der Tabelle 3 ersichtlich ist, umfasst das Spektrum an Tieren Subjekte der Rassen Ragdoll (27 Exemplare), Britisch Kurzhaar (6 Exemplare), Britisch Langhaar (1 Exemplar), Sibirische Katze und die nah verwandte Point-Variante Neva Masquarade (2 und 3 Exemplare), Maine-Coon (4 Exemplare), Heilige Birma (5 Exemplare), Schottische Faltohrkatze (3 Exemplare), Türkisch Angora (8 Exemplare), Perser (1 Exemplar), Abessinier (2 Exemplare) und Europäisch Kurzhaar (3 Exemplare). Außerdem wurden weiterhin sechs Proben von Katzen unbekannter Rasse untersucht, welche als Mischlinge bezeichnet werden.

**Tabelle 3: Vollständig sequenzierte Katzen geordnet nach Rassen und Blutgruppen**

| **Rasse** | **Anzahl Testsubjekte** | **Blutgruppen** |
|---|---|---|
| Ragdoll | 27 | 1 A; 14 B; 12 C |
| Britisch Kurzhaar | 6 | 1 A; 4 B; 1 C |
| Britisch Langhaar | 1 | 1 B |
| Sibirische Katze | 2 | 1 A; 1 C |
| Neva Masquarade | 3 | 1 A; 2 B |
| Maine-Coon | 4 | 2 A; 2 B |
| Heilige Birma | 5 | 1 A; 4 B |
| Schottische Faltohrkatze | 3 | 1 A; 2 B |
| Türkisch Angora | 8 | 4 A; 4 B |
| Perser | 1 | 1 A |
| Abessinier | 2 | 1 A; 1 B |
| Mischlinge | 6 | 6 C |
| Europäisch Kurzhaar | 3 | 3 B |

Die Sequenzen werden mithilfe des Basic Local Alignment and Search Tools (BLAST) mit der Sequenz einer Katze als Referenzgenom aus der NCBI verglichen, um mögliche Mutationen bzw. SNPs feststellen zu können, die sich als Ursache des AB-Blutgruppensystems herausstellen könnten. Bei der Referenz-Katze handelt es sich um eine Katze der Blutgruppe A und der Rasse Abessinier, zur Verfügung gestellt vom International Cat Genome Sequencing Consortium.

### Beispiel 6: Sequenzierungsergebnisse verschiedener Rassekatzen

In der nachfolgenden Tabelle 4, welche sich aus den Tabellen 4a und 4b zusammensetzt, sind die im Rahmen der vorliegenden Erfindung gemachten Sequenzierungsergebnisse für die verschiedenen Rassekatzen und die untersuchten Mischlinge zusammengefasst. Dabei gilt, dass die Buchstaben C, T, A und G die entsprechende in der Sequenzierung nachgewiesene Nukleinbasen beschreiben. Die Buchstaben N und P stehen für nichtanwesend (N) und anwesend (P), wohingegen x kennzeichnet, dass die Sequenzierung nicht erfolgreich war.

### Tabelle 4: Darstellung der Sequenzierungsergebnisse verschiedener Rassekatzen

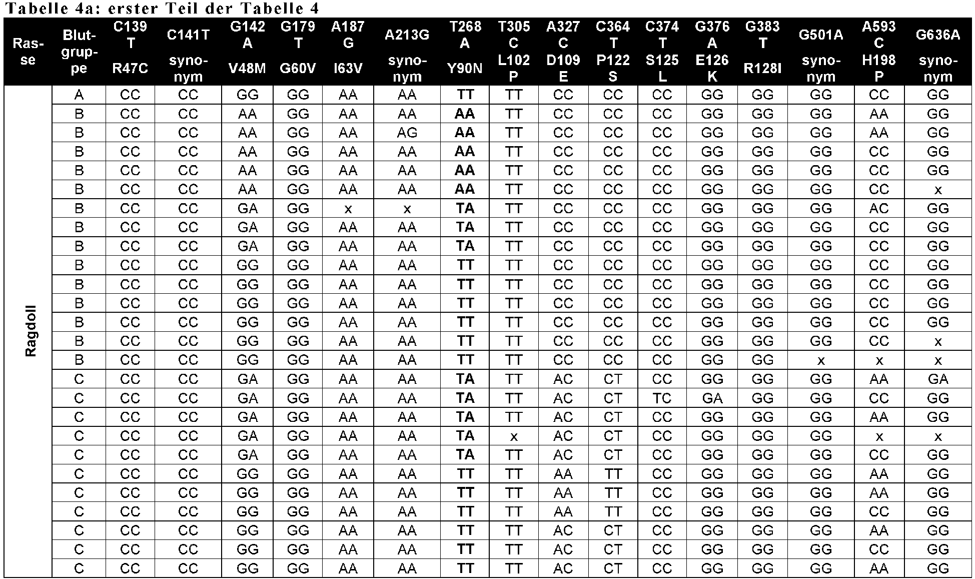

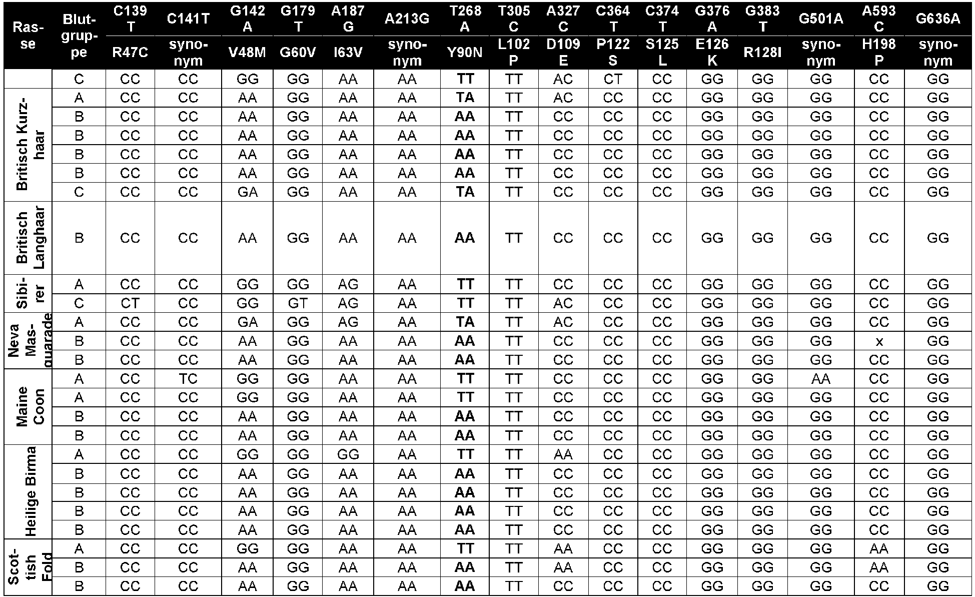

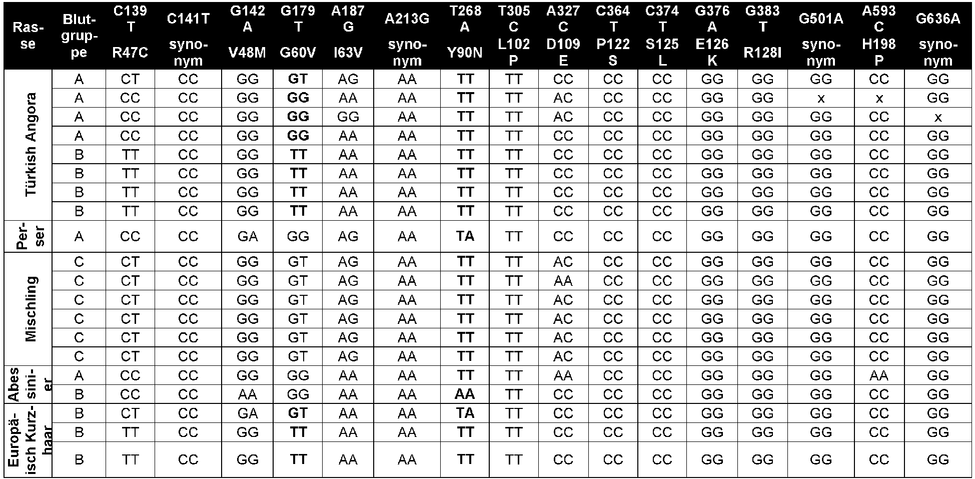

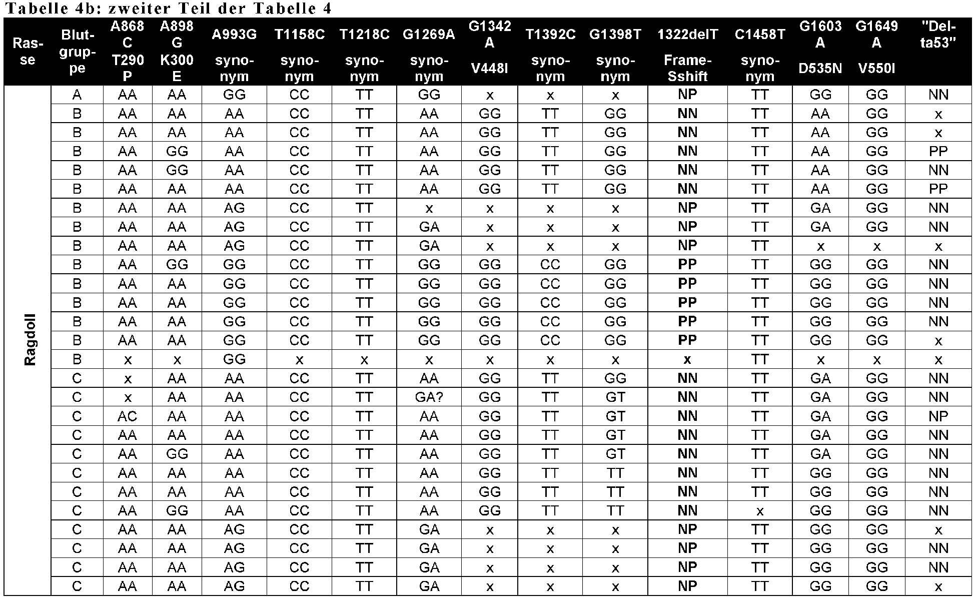

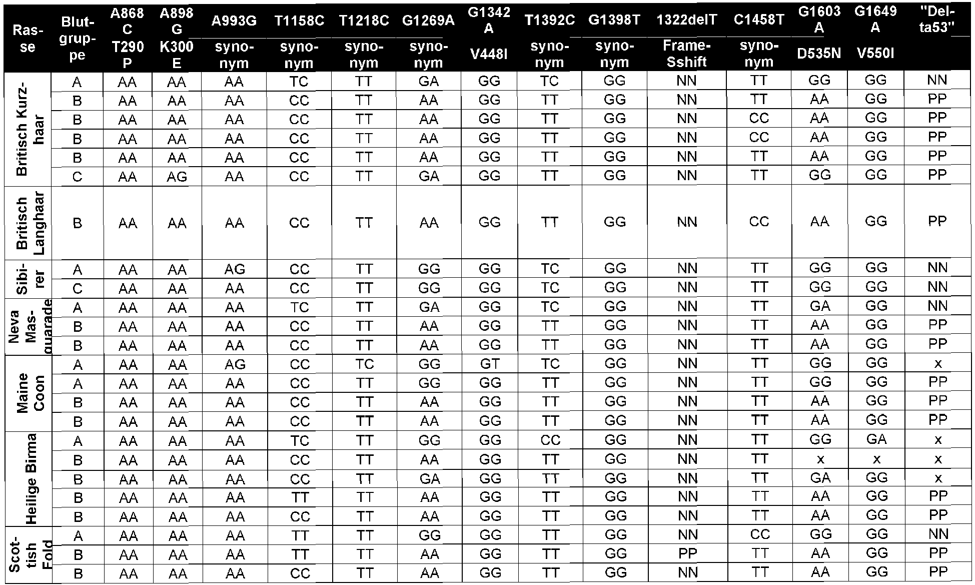

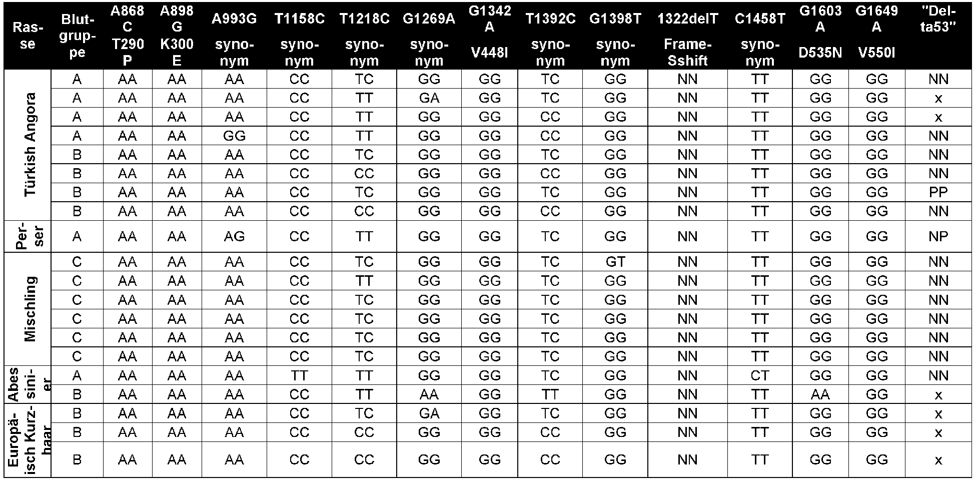

### Beispiel 7: Ergebnisse der SNP-Assays

In der nachfolgenden Tabelle 5 sind die im Rahmen der vorliegenden Erfindung gemachten Ergebnisse zu Diplotypen bei den SNP-Assays für die Mutationen 179G>T, 268T>A und die Deletion bei 1322delT zusammengefasst. Zudem werden bei jeder Katze die Mutationen Delta-53 und 142G>A untersucht. Dabei gilt, dass die Buchstaben N und b die entsprechenden Allele bezeichnen, wobei N für das Allel A (wildtypisch) und b für das Allel b steht. Mit einem x bezeichnete Ergebnisse kennzeichnen, dass der SNP-Assay nicht erfolgreich durchgeführt werden konnte, wohingegen mit einem "-" bezeichnete weitere Assays für die entsprechende Katze nicht durchgeführt wurden.

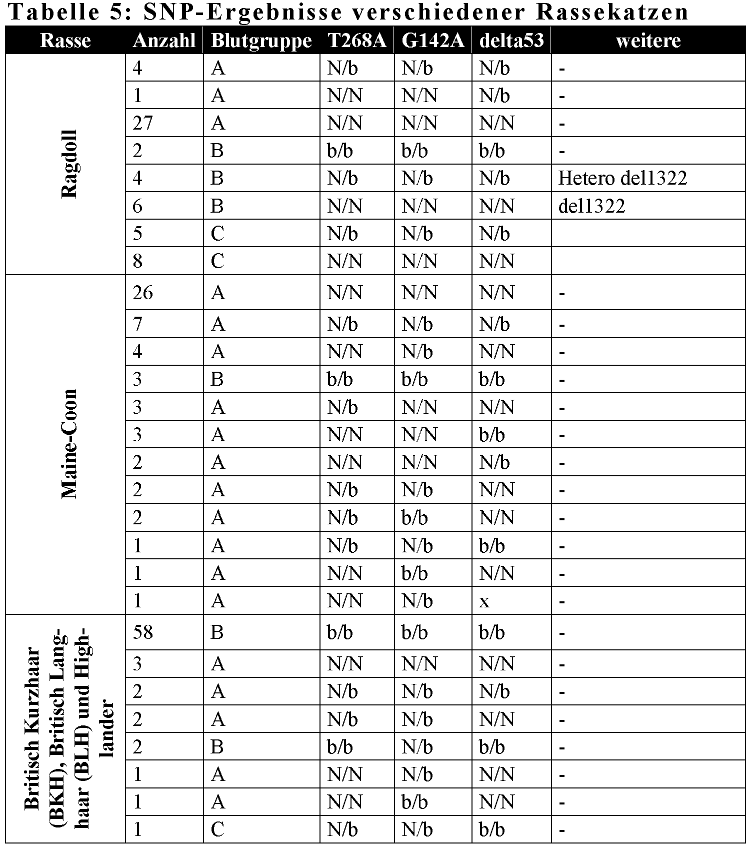

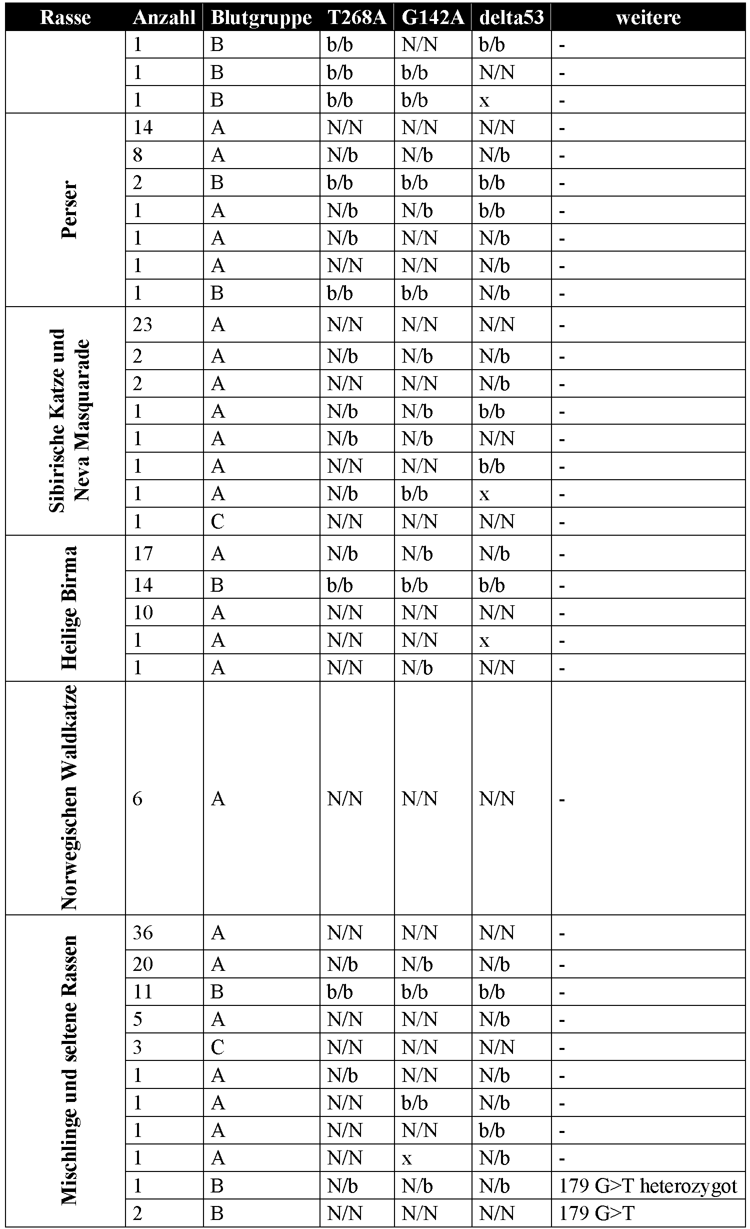

## Patentansprüche

1. Verfahren zur Bestimmung der Blutgruppe einer Katze im AB-Blutgruppensystem, welches die folgenden Schritte umfasst:
a) Ermitteln der Anwesenheit oder Abwesenheit von drei Mutationen in einem von beiden Allelen oder in beiden Allelen des felinen Gens *Cytidin-Monophosphat-N-Acetylneuraminsäure-Hydroxylase* (*CMAH*) in einer biologischen Probe, wobei die Mutationen eine G zu T Substitution an Position 179, eine T zu A Substitution an Position 268 und eine T-Deletion an Position 1322 sind; und
b) Bestimmen der Blutgruppe der Katze, wobei die Anwesenheit von einer der Mutationen in einem von beiden Allelen und/oder die Abwesenheit von mindestens einer der Mutationen in beiden Allelen indikativ für die Blutgruppe A ist oder wobei die Anwesenheit von mindestens einer der Mutationen in beiden Allelen und/oder die Anwesenheit von mindestens zwei der Mutationen in einem von beiden Allelen indikativ für die Blutgruppe B ist.

2. Verfahren zur Bestimmung der Blutgruppe einer Katze im AB-Blutgruppensystem, welches die folgenden Schritte umfasst:
a) Ermitteln der Anwesenheit oder Abwesenheit von vier Mutationen in einem von beiden Allelen oder in beiden Allelen des felinen Gens *Cytidin-Monophosphat-N-Acetylneuraminsäure-Hydroxylase* (*CMAH*) in einer biologischen Probe, wobei die Mutationen eine G zu T Substitution an Position 179, eine T zu A Substitution an Position 268, eine C zu T Substitution an Position 364 und eine T-Deletion an Position 1322 sind; und
b) Bestimmen der Blutgruppe der Katze, wobei die Anwesenheit von der Mutation an Position 364 in beiden Allelen oder von der Mutation an Position 364 in einem Allel und von mindestens einer der Mutationen ausgewählt aus der Gruppe umfassend die Positionen 179, 268 und 1322 in einem von beiden Allelen oder in beiden Allelen indikativ für die Blutgruppe C ist.

3. Verfahren nach einem der vorhergehenden Ansprüche, wobei das Ermitteln umfasst:
a) Amplifizieren einer Nukleinsäure, wobei die Nukleinsäure ausgewählt ist aus der Gruppe umfassend
i) eine Nukleinsäure-Sequenz wie in SEQ ID NO: 1 gezeigt; und
ii) eine Nukleinsäure-Sequenz, welche ein Polypeptid codiert, das eine Aminosäure-Sequenz wie in SEQ ID NO: 2 gezeigt aufweist; und
iii) eine Nukleinsäure-Sequenz, welche ein Polypeptid codiert, das mindestens 50 % identisch zu einem Polypeptid ist, das von den Nukleinsäure-Sequenzen aus i) oder ii) codiert wird, wobei das Polypeptid die feline CMAH ist; und
iv) eine Nukleinsäure-Sequenz für ein Fragment einer Nukleinsäure aus i), ii) oder iii), wobei das Fragment ein Polypeptid codiert, wobei das Polypeptid die feline CMAH ist; und
b) Detektieren der amplifizierten Nukleinsäure-Sequenz.

4. Verfahren nach einem der vorhergehenden Ansprüche, wobei die Katze eine Großkatze, eine Kleinkatze, eine Hauskatze, eine Rassekatze und/oder ein Mischling ist.

5. Verfahren nach einem der vorhergehenden Ansprüche, wobei die biologische Probe eine Körperflüssigkeit, eine biologische Flüssigkeit, ein Gewebe, eine Zellprobe, ein Gewebeschnitt und/oder ein Gefrierschnitt ist.

## Claims

1. A method for identifying the blood group of a cat in the AB blood group system, the method comprising the following steps:
a) determining the presence or absence of three mutations in one of the two alleles or in both alleles of feline gene *Cytidine Monophosphate-N-Acetylneuraminic Acid Hydroxylase* (*CMAH*) in a biological sample, the mutations consisting in a G to T substitution at position 179, a T to A substitution at position 268, and a T deletion at position 1322; and
b) identifying the blood group of the cat, the presence of one of the mutations in one of the two alleles and/or the absence of at least one of the mutations in both alleles being indicative of blood group A, or the presence of at least one of the mutations in both alleles and/or the absence of at least two of the mutations in one of the two alleles being indicative of blood group B.

2. A method for identifying the blood group of a cat in the AB blood group system, the method comprising the following steps:
a) determining the presence or absence of four mutations in one of the two alleles or in both alleles of feline gene *Cytidine Monophosphate-N-Acetylneuraminic Acid Hydroxylase* (*CMAH*) in a biological sample, the mutations consisting in a G to T substitution at position 179, a T to A substitution at position 268, a C to T substitution at position 364, and a T deletion at position 1322; and
b) identifying the blood group of the cat, the presence of the mutation at position 364 in both alleles or of the mutation at position 364 in one allele and of at least one of the mutations selected from the group comprising positions 179, 268, and 1322 in one of the two alleles or in both alleles being indicative of blood group C.

3. The method according to any one of the preceding claims, the determining step comprising:
a) amplifying a nucleic acid, the nucleic acid being selected from the group comprising
i) a nucleic acid sequence as shown in SEQ ID NO: 1; and
ii) a nucleic acid sequence encoding a polypeptide having an amino acid sequence as shown in SEQ ID NO: 2; and
iii) a nucleic acid sequence encoding a polypeptide that is at least 50 % identical to a polypeptide encoded by the nucleic acid sequences of i) or ii), the polypeptide being feline CMAH; and
iv) a nucleic acid sequence for a fragment of a nucleic acid of i), ii) or iii), the fragment encoding a polypeptide, the polypeptide being feline CMAH; and
b) detecting the amplified nucleic acid sequence.

4. The method according to any one of the preceding claims, wherein the cat is a big cat, a small cat, a domestic cat, a pedigree cat, and/or a mixed breed.

5. The method according to any one of the preceding claims, wherein the biological sample is a body fluid, a biological fluid, tissue, a cell sample, a tissue section, and/or a frozen section.

## Revendications

1. Procédé d'identification du groupe sanguin d'un chat dans le système de groupes sanguins AB, le procédé comprenant les étapes suivantes :
a) déterminer la présence ou l'absence de trois mutations dans un des deux allèles ou dans les deux allèles du gène félin *cytidine monophosphate-acide N-acétylneuraminique hydroxylase* (*CMAH*) dans un échantillon biologique, les mutations étant une substitution G à T en position 179, une substitution T à A en position 268 et une délétion T en position 1322 ; et
b) identifier le groupe sanguin du chat, la présence d'une des mutations dans un des deux allèles et/ou l'absence d'au moins une des mutations dans les deux allèles indiquant le groupe sanguin A ou la présence d'au moins une des mutations dans les deux allèles et/ou l'absence d'au moins deux des mutations dans un des deux allèles indiquant le groupe sanguin B.

2. Procédé d'identification du groupe sanguin d'un chat dans le système de groupes sanguins AB, le procédé comprenant les étapes suivantes :
a) déterminer la présence ou l'absence de quatre mutations dans un des deux allèles ou dans les deux allèles du gène félin *cytidine monophosphate-acide N-acétylneuraminique hydroxylase* (*CMAH*) dans un échantillon biologique, les mutations étant une substitution G à T en position 179, une substitution T à A en position 268, une substitution C à T en position 364 et une délétion T en position 1322 ; et
b) identifier le groupe sanguin du chat, la présence de la mutation en position 364 dans les deux allèles ou de la mutation en position 364 dans un allèle et d'au moins une des mutations sélectionnées dans le groupe comprenant les positions 179, 268 et 1322 dans un des deux allèles ou dans les deux allèles indiquant le groupe sanguin C.

3. Procédé selon l'une quelconque des revendications précédentes, dans lequel l'étape de détermination comprend les étapes consistant à :
a) amplifier un acide nucléique, l'acide nucléique étant sélectionné dans le groupe comprenant
i) une séquence d'acide nucléique comme indiquée par SEQ ID NO : 1 ; et
ii) une séquence d'acide nucléique codant pour un polypeptide ayant une séquence d'acides aminés comme indiquée par SEQ ID NO : 2 ; et
iii) une séquence d'acide nucléique codant pour un polypeptide qui est au moins 50 % identique à un polypeptide encodé par les séquences d'acides nucléiques de i) ou ii), le polypeptide étant la CMAH féline ; et
iv) une séquence d'acide nucléique pour un fragment d'un acide nucléique de i), ii) ou iii), le fragment codant pour un polypeptide, le polypeptide étant la CMAH féline ; et
b) détecter la séquence d'acide nucléique amplifiée.

4. Procédé selon l'une quelconque des revendications précédentes, dans lequel le chat est un grand félin, un petit félin, un chat domestique, un chat de race et/ou un chat de race mixte.

5. Procédé selon l'une quelconque des revendications précédentes, dans lequel l'échantillon biologique est un fluide corporel, un fluide biologique, un tissu, un échantillon cellulaire, une coupe tissulaire et/ou une coupe congelée.
